# EUROPEAN PATENT SPECIFICATION

(11) **EP 0 665 229 B1**
(45) Date of publication and mention of the grant of the patent: **17.11.1999**
(21) Application number: 95101370.5
(22) Date of filing: 01.02.1995
(51) Int. Cl.: C07D 473/02, A61K 31/52

(54) **Process for the production of nucleic acid base derivatives**
Verfahren zur Herstellung von Nukleinsäurebasenderivaten
Procédé pour la production de dérivés d'une base d'acide nucléique

(30) Priority: 01.02.1994 JP 1061794
(43) Date of publication of application: 02.08.1995
(73) Proprietor: Ajinomoto Co., Inc., Tokyo 104 (JP)
(72) Inventor: Takamatsu, Satoshi, c/o Central Research Lab., Kawasaki-shi, Kanagawa-ken (JP); Izawa, Kunisuke, c/o Central Research Lab., Kawasaki-shi, Kanagawa-ken (JP); Uchida, Yumiko, c/o Central Research Lab., Kawasaki-shi, Kanagawa-ken (JP); Ineyama, Takashi, c/o Central Research Lab., Kawasaki-shi, Kanagawa-ken (JP)
(74) Representative: Strehl Schübel-Hopf & Partner

(56) References cited:
- EP-A- 0 099 493
- EP-A- 0 308 065
- EP-A- 0 596 542

## Description

### Field of the Invention

This invention relates to a process for the production of valacyclovir which is a derivative of acyclovir used worldwide as an antiviral agent and which is currently deemed to be useful for the treatment of diseases caused by the cytomegalovirus and the like.

### Prior Art

9-[(2-Hydroxyethoxy)methyl]guanine which is known as acyclovir has strong antiviral activity towards the herpes virus. However, since the acyclovir is less water-soluble and has low oral absorption, a large amount of a medication has to be administered in the treatment process.

In order to improve these properties, the production of various derivatives of acyclovir has been attempted, and the synthesis of, for example, O-alkyl derivatives of acyclovir, O-valeryl derivatives of acyclovir, O-glycine and alanine derivatives of acyclovir (Japanese Patent Publication No. 4-990), and O-valine and isoleucine derivatives of acyclovir (Japanese Laid-Open Patent Application (Kokai) No. 64-68,373) has been reported so far. Among the attempts of synthesizing these various derivatives of acyclovir, an amino acid ester or the like, especially an ester of L-valine is deemed to be the best from the standpoint of oral absorption or the like [Antiviral Chemistry & Chemotherapy (1992) 3(3), pp. 157-164].

Under the circumstances, an ester of an amino acid and an antiviral agent having a pseudo-sugar moiety such as acyclovir has been in increasing demand. However, the processes of its synthesis are still unsatisfactory. According to Japanese Laid-Open Patent Application (Kokai) No. 64-68,373 and Antiviral Chemistry & Chemotherapy (1992) 3(3), pp. 157-164, when an ester of an amino acid such as L-valine and acyclovir is synthesized, an N-protected amino acid and acyclovir are used as raw materials and condensed with dicyclohexylcarbodiimide as a condensation agent. However, since the solubility of the formed ester of N-protected amino acid and acyclovir is low, several recrystallizations or purification through silica-gel column is needed to remove dicyclohexylurea which forms as a by-product. Accordingly, this process is not industrially feasible.

### Problems to be Solved by the Invention

This invention is to provide a process which is industrially useful for the synthesis of an ester of an amino acid and a nucleoside having a pseudo-sugar moiety.

### Means for Solving the Problems

The present inventors have assiduously investigated in order to develop a novel process for the production of an ester of amino acid and a nucleoside having an amino group in a nucleic acid base moiety, especially a nucleoside having a purine ring in the base moiety and an amino group in the 2-position, wherein an ester is synthesized by the condensation of an amino acid and a sugar or a pseudo-sugar moiety. They have consequently found that when an amino acid and a sugar or pseudo-sugar moiety are condensed, acylation of the amino group of the nucleic acid base moiety facilitates the purification after the completion of the condensation. This finding has led to the completion of this invention.

That is, this invention is to provide a process for the production of a compound represented by formula (1) 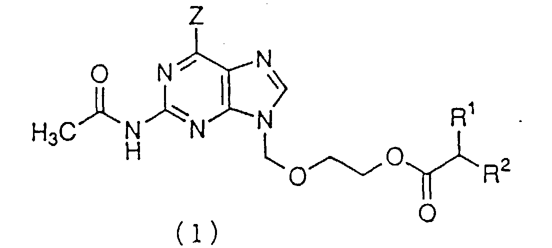 wherein Z denotes an optionally protected hydroxyl group, an optionally protected amino group, iodine, chlorine or hydrogen, R¹ denotes hydrogen, an alkyl group having from 1 to 10 carbon atoms, an aralkyl group, an optionally protected aminoalkyl group or an optionally protected carboxylalkyl group, and R² denotes an optionally protected amino group,
which comprises reacting a compound represented by formula (2) 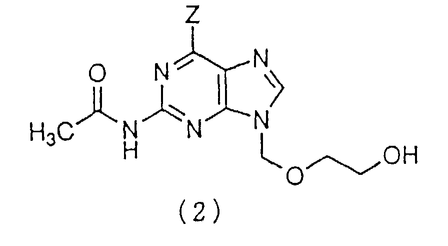 wherein Z denotes an optionally protected hydroxyl group, an optionally protected amino group, iodine, chlorine or hydrogen,
with a compound represented by formula (3) 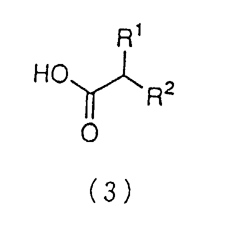 wherein R¹ denotes hydrogen, an alkyl group having 1 to 10 carbon atoms, an aralkyl group, an optionally protected aminoalkyl group or an optionally protected carboxylalkyl group, and R² denotes an optionally protected amino group;
wherein the reaction is conducted in the presence of dicyclohexylcarbodiimide; and
wherein dicyclohexylurea which is formed as a by-product of the reaction is removed by filtration. Further, this invention is to provide a compound represented by formula (1) 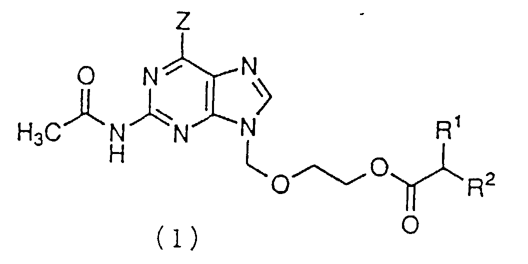 wherein Z is an optionally protected hydroxyl group, an optionally protected amino group, iodine, chlorine or hydrogen, R¹ denotes hydrogen, an alkyl group having from 1 to 10 carbon atoms, an aralkyl group, an optionally protected aminoalkyl group or an optionally protected carboxylalkyl group, and R² denotes an optionally protected amino group,
which is obtainable by the above-mentioned reaction; and 2-[(2-acetylamino-1,6-dihydro-6-oxo-9H-purin-9-yl)methoxy]ethyl N-benzyloxycarbonyl-L-valinate represented by formula (4) 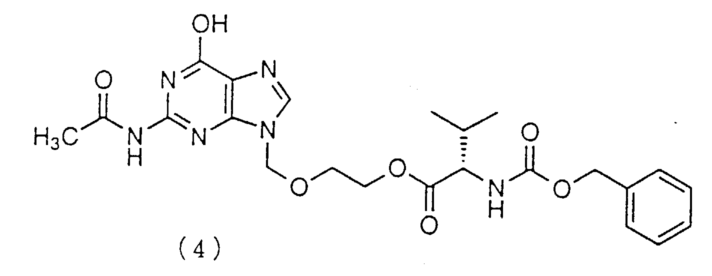 which is a typical compound.

The compound of formula (2) which is used in this invention can be obtained by reacting the corresponding compound, as a diacyl compound, represented by formula (5) 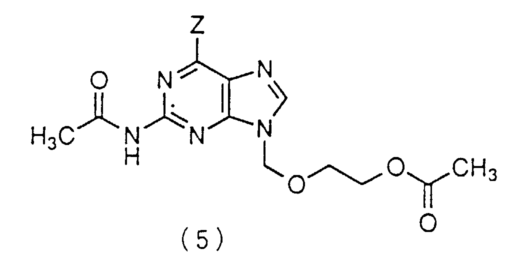 wherein Z is as defined above,
with a base. Examples of the base include hydroxylamine, ammonia, their salts, primary to tertiary amines, their salts, quaternary ammonium salts, metal alkoxides such as sodium methoxide and potassium methoxide, and alkali solutions such as sodium hydroxide and lithium hydroxide.

The compound of formula (5) can be obtained by reacting a compound free of an N- or O-acetyl group with, for example, acetic anhydride in an acetic acid solvent in the presence of an acid catalyst such as p-toluenesulfonic acid. When Z is the hydroxyl group in formula (5), the compound can be obtained by, for example, a process described in Japanese Laid-Open Patent Application (Kokai) No. 5-78,329.

When Z is the hydroxyl group in formula (2), the compound can be obtained by, for example, a process described in Japanese Laid-Open Patent Application (Kokai) No. 63-107,981.

The compound of formula (3) which is used in this invention is an amino acid in which an amino group is optionally protected. The amino acid may be an L-isomer, a D-isomer or a racemic compound.

Examples of the amino acid include glycine, alanine, valine, leucine, isoleucine, serine, threonine, proline, aspartic acid, asparagine, glutamic acid, glutamine, histidine, lysine, ornithine, arginine, phenylalanine, tyrosine and tryptophan. Other amino acids are also available.

It is advisable that the amino group or the carboxyl group in the side chain of the amino acid be protected with a protective group. Examples of the protective group of the amino group include urethane-type protective groups such as a benzyloxycarbonyl group, a tert-butoxycarbonyl group and a 9-fluorenylmethyloxycarbonyl group; monocarboxylic acid-acyl-type protective groups such as a formyl group, an acetyl group, a trifluoroacetyl group and a benzoyl group; dicarboxylic acid-acyl-type protective groups such as a phthaloyl group; sulfonic acid-acyl-type protective groups such as a tosyl group; and alkyl-type protective groups such as a triphenylmethyl group and a benzyl group. Examples of the protective group of the carboxyl group include ester-type protective groups such as a methyl ester group, an ethyl ester group, a benzyl ester group, a substituted benzyl ester group, a tert-butyl ester group, a cyclopentyl ester group, a cyclohexyl ester group and a phenacyl ester group.

Specific examples of the compound in which the side chain of the amino acid is protected include benzyloxycarbonyl-L-glycine, benzyloxycarbonyl-L-alanine, benzyloxycarbonyl-L-valine, benzyloxycarbonyl-L-leucine, benzyloxycarbonyl-L-isoleucine, benzyloxycarbonyl-L-serine, benzyloxycarbonyl-L-threonine, benzyloxycarbonyl-L-proline, ω-benzyl benzyloxycarbonyl-L-aspartate, ω-tert-butyl benzyloxycarbonyl-L-aspartate, benzyloxycarbonyl-L-asparagine, ω-benzyl benzyloxycarbonyl-L-glutamate, ω-tert-butyl benzyloxycarbonyl-L-glutamate,benzyloxycarbonyl-L-glutamine, benzyloxycarbonyl-L-histidine, α-benzyloxycarbonyl-ω-tert-butoxycarbonyl-L-lysine, α,ω-dibenzyloxycarbonyl-L-lysine, α-benzyloxycarbonyl-ω-tert-butoxycarbonyl-L-ornithine, α,ω-dibenzyloxycarbonyl-L-ornithine, α-benzyloxycarbonyl-ω -p-toluenesulfonyl-L-arginine, α-benzyloxycarbonyl-ω-nitro-L-arginine, benzyloxycarbonyl-L-phenylalanine, benzyloxycarbonyl-L-tyrosine, benzyloxycarbonyl-O-benzyl-L-tyrosine, benzyloxycarbonyl-L-tryptophan, tert-butoxycarbonyl-L-glycine, tert-butoxycarbonyl-L-alanine, tert-butoxycarbonyl-L-valine, tert-butoxycarbonyl-L-leucine, tert-butoxycarbonyl-L-isoleucine, tert-butoxycarbonyl-L-serine, tert-butoxycarbonyl-L-threonine, tert-butoxycarbonyl-L-proline, tert-butoxycarbonyl-L-aspartic acid, ω-benzyl tert-butoxycarbonyl-L-aspartate, ω-cyclohexyl tert-butoxycarbonyl-L-aspartate, tert-butoxycarbonyl-L-asparagine, ω-benzyl tert-butoxycarbonyl-L-glutamate, ω-tert-butyl tert-butoxycarbonyl-L-glutamate, tert-butoxycarbonyl-L-glutamine, tert-butoxycarbonyl-L-histidine, α-tert-butoxycarbonyl-ω-trifluoroacetyl-L-lysine, α-tert-butoxycarbonyl-ω-p-toluenesulfonyl-L-lysine, α-tert-butoxycarbonyl-ω-benzyloxycarbonyl-L-lysine, α-tert-butoxycarbonylω-benzyloxycarbonyl-L-ornithine, α,ω-di-tert-butoxycarbonyl-L-ornithine, α-tert-buthoxycarbonyl-ω-p-toluene sulfonyl-L-arginine, α-tert-butoxycarbonyl-ω-nitro-L-arginine, tert-butoxycarbonyl-L-phenylalanine, tert-butoxycarbonyl-L-tyrosine, benzyloxycarbonyl-O-benzyl-L-tyrosine, and tert-butoxycarbonyl-L-tryptophan.

A suitable condensation agent is used to produce the compound of formula (1) by the reaction of compounds of formulas (2) and (3). Specific examples of the condensation agent include dicyclohexylcarbodiimide, water-soluble carbodiimide and carbonyldiimidazole. The compound of formula (1) can be produced in a solvent. Examples of the solvent include dimethylformamide, dimethyl sulfoxide, ethyl acetate, methylene chloride, acetonitrile, toluene and tetrahydrofuran. A base catalyst may be optionally used in the above-mentioned reaction. Examples of the base catalyst include 4-dimethylaminopyridine, triethylamine and pyridine. An additive may be optionally used in the above-mentioned reaction. Examples of the additive include N-hydroxysuccinimide and 1-hydroxybenzotriazole. The reaction temperature can be selected to be between -20°C and 40°C.

The compound of formula (1) formed by the reaction has greatly improved physical properties such as solubility in an organic solvent compared to the corresponding compound represented by formula (6) 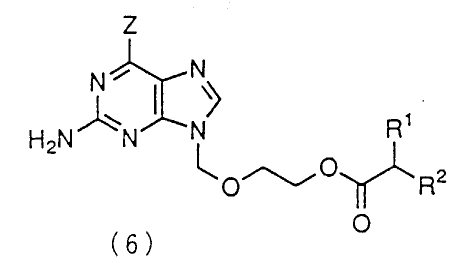 wherein R¹, R² and Z are as defined above.
For example, the solubility in ethyl acetate at room temperature of 2-[(2-acetylamino-1,6-dihydro-6-oxo-9H-purin-9-yl)methoxy]ethyl N-benzyloxycarbonyl-L-valinate (hereinafter simply referred to as "NAcZVA") represented by formula (4) 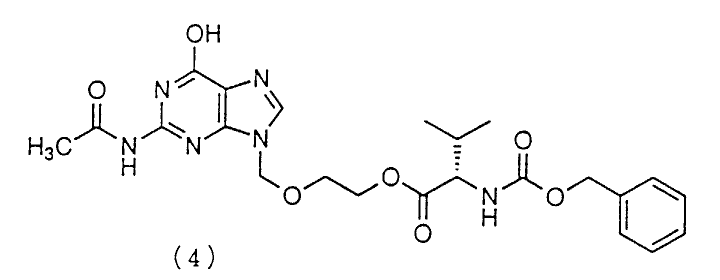 is 0.54 g/dl. While that of the corresponding compound, 2-[(2-amino-1,6-dihydro-6-oxo-9H-purin-9-yl)methoxy]ethyl N-benzyloxycarbonyl-L-valinate (hereinafter simply referred to as "ZVA") represented by formula (7) 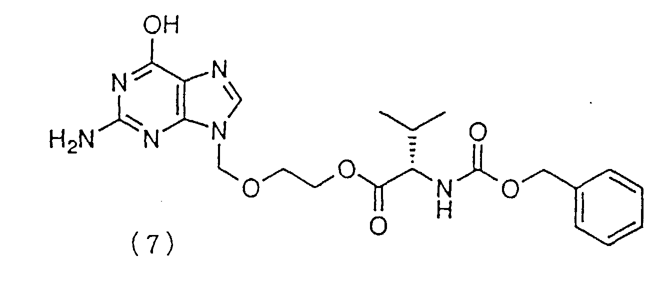 is 0.04 g/dl. Thus, the solubilities of NAcZVA is over 10 times that of ZVA. Further, NAcZVA has high solubilities in acetonitrile and in methylenechloride at room temperature 9.6 g/dl and 52.7 g/dl respectively. Accordingly, if a solvent, the solvent concentration and the temperature for the reaction are suitably selected, the purification of the compound is completed only by treatment by an ordinary solvent extraction method or by the filtration of a sparingly soluble precipitate, such as dicyclohexylurea, after the completion of the reaction.

The thus-obtained compound of formula (2) can be converted into the ester of an amino acid and a nucleoside which is a final compound by deprotecting the N-acetyl group of the purine base moiety and the N-protecting group of the amino acid moiety in an optional order. In order to deprotect the N-acetyl group of the purine base moiety, the compound may be reacted with a base such as ammonia or tertiary amines or their salts in a hydrous or an anhydrous alcohol having 1 to 6 carbon atom(s) . The reaction temperature can be optionally selected from room temperature to the refluxing temperature of the solvent. The N-protecting group of the amino acid moiety may be deprotected according to a general method. For example, the deprotection of the benzyloxycarbonyl group or tert-butoxycarbonyl group in the amino acid ester of acyclovir is described in Antiviral Chemistry & Chemotherapy (1992) 3(3), pp. 157-164.

### Examples

This invention is illustrated in more detail by the following Examples.

### Example 1

### Synthesis of 2-[(2-acetylamino-1,6-dihydro-6-oxo-9H-purin-9-yl)methoxy]ethyl N-[(benzyloxy)carbonyl]-L-valinate from 9-[(2-hydroxyethoxy)methyl]-N²-acetylguanine:

One hundred milliliters of dimethylformamide were added to 2.67 g of 9-[(2-hydroxyethoxy)methyl]-N²-acetylguanine, and the mixture was heat-dissolved at 60°C. The solution was cooled to room temperature, and 3.27 g of N-benzyloxycarbonyl-L-valine, 0.17 g of 4-dimethylaminopyridine and 3.30 g of dicyclohexylcarbodiimide were added thereto. The mixed solution was stirred at room temperature for 118 hours. To the reaction mixture were further added N-benzyloxycarbonyl-L-valine, 4-dimethylaminopyridine and dicyclohexylcarbodiimide in the same amounts. The mixture was stirred at room temperature for 17 hours. The white solid precipitate was filtered from the reaction mixture and washed with a small amount of dimethylformamide. The filtrate was analyzed by liquid chromatography. Consequently, 2-[(2-acetylamino-1,6-dihydro-6-oxo-9H-purin-9-yl)methoxy]ethyl N-[(benzyloxy)carbonyl]-L-valinate was obtained in a yield of 99.7 %. Further, the filtrate was concentrated under reduced pressure, and the obtained oily substance was dissolved in 100 ml of ethyl acetate and washed with 50 ml of water and with 10 ml of a saturated NaCl aqueous solution. After the aqueous layer was extracted with 100 ml of ethyl acetate, the organic layers were collected, dried over anhydrous sodium sulfate, then filtered and allowed to stand at room temperature. One hour later, the precipitated crystals were filtered. The filtrate was further allowed to stand overnight at room temperature, and the precipitated crystals were combined to obtain a sample for the analysis of 2-[(2-acetylamino-1,6-dihydro-6-oxo-9H-purin-9-yl)methoxy]ethyl N-[(benzyloxy)carbonyl]-L-valinate. Nuclear magnetic resonance analysis (¹H, DMSO-D6)
δ 0.81(6H,d,iPr-Me), 1.93(1H,m,iPr-CH), 2.17 (3H,s,ACV-NAc),3.69(2H,m,ACV-3'H),3.86(1H,m, Val- H),4.07-4.25 (2H,m,ACV-4'H), 5.02(2H,s, Cbz-CH2), 5.47 (2H,s,ACV-1'H), 7.29-7.40 (5H,m, Cbz-Ph), 7.64 (1H,d,Val-NH), 8.12 (1H,s,ACV-8H), 11.80-12.20 (2H,b, ACV-1H+2NH)
Mass spectrum analysis (FAB mode) M calculated (M+H⁺ C₂₃H₂₉O₇N₆): 501. 1098 found: 501. 2111

### Example 2

### Synthesis of 2-[(2-amino-1,6-dihydro-6-oxo-9H-purin-9-yl)methoxy]ethyl N-[(benzyloxy)carbonyl]-L-valinate from 9-[(2-hydroxyethoxy)methyl]-N²-acetylguanine:

Fifty milliliters of dimethylformamide were added to 2.67 g of 9-[(2-hydroxyethoxy)methyl]-N²-acetylguanine, and the mixture was cooled to 0°C. To the mixture were added 2.52 g of N-benzyloxycarbonyl-L-valine, 0.12 g of 4-dimethylaminopyridine and 2.29 g of dicyclohexylcarbodiimide. The thus-obtained mixture was stirred at 0°C for 3 hours and then at room temperature for 13 hours. To the reaction mixture were further added N-benzyloxycarbonyl-L-valine, 4-dimethylaminopyridine and dicyclohexylcarbodiimide in the same amounts, followed by stirring them at room temperature for 48 hours. The precipitate was filtered from the reaction mixture, and the filtrate was concentrated under reduced pressure to obtain an oily substance. The oily substance was dissolved in 200 ml of ethyl acetate. The organic layer was washed once with 100 ml of a 5 % potassium hydrogensulfate aqueous solution and twice with 100 ml of a saturated NaCl aqueous solution, dried over anhydrous magnesium sulfate, and concentrated under reduced pressure to obtain an oily substance. The oily substance was analyzed by liquid chromatography. Consequently, 2-[(2-acetylamino-1,6-dihydro-6-oxo-9H-purin-9-yl)methoxy]ethyl N-[(benzyloxy) carbonyl]-L-valinate was obtained in a yield of 81.1 %.

The oily substance was dissolved in 40 ml of ethanol, and 4.05 g of triethylamine were added to the solution, followed by refluxing the mixed solution for 7 hours. After the completion of the reaction, the mixture was analyzed by liquid chromatography. As a result, the intended 2-[(2-amino-1,6-dihydro-6-oxo-9H-purin-9-yl)methoxy]ethyl N-[(benzyloxy)carbonyl]-L-valinate was obtained from 9-[(2-hydroxyethoxy)methyl]-N²-acetylguanine in a yield of 77.2 %. The reaction mixture was further cooled to 0°C, and the precipitated crystals were filtered and washed with a small amount of ethanol. The thus-obtained crystals were dried at 50°C under reduced pressure to obtain 3.36 g of crystals of 2-[(2-amino-1,6-dihydro-6-oxo-9H-purin-9-yl)methoxy]ethyl N-[(benzyloxy)carbonyl]-L-valinate. The yield of this compound from 9-[(2-hydroxyethoxy)methyl]-N²-acetylguanine was 73.3 %.
Nuclear magnetic resonance analysis (¹H, DMSO-D6)
δ 0.83 (6H, d, iPr-Me), 1.96 (1H, m, iPr-CH), 3.66(2H, m,ACV-3'H),3.90(1H,m,Val-H), 4.10-4.20 (2H, m, ACV-4'H), 5.03 (2H, s, Cbz-CH2), 5.35 (2H, s, ACV-1'H), 6.51 (2H, s, ACV-2NH2), 7.25-7.45 (5H, m, Cbz-Ph), 7.67(1H, d, Val-NH), 7.81 (1H, s, ACV-8H), 10.63 (1H, s, ACV-1H)

### Example 3

### Synthesis of 2-[(2-amino-1,6-dihydro-6-oxo-9H-purin-9-yl)methoxy]ethyl N-[(benzyloxy)carbonyl]-L-valinate from 9-[(2-hydroxyethoxy)methyl]-N²-acetylguanine:

Fifty milliliters of acetonitrile were added to 2.67 g of 9-[(2-hydroxyethoxy)methyl]-N²-acetylguanine, and the mixture was cooled to 0°C. To the mixture were added 2.52 g of N-benzyloxycarbonyl-L-valine, 0.12 g of 4-dimethylaminopyridine and 2.29 g of dicyclohexylcarbodiimide. The mixture was stirred at the freezing point for 3 hours and then at room temperature for 8.5 hours. To the reaction mixture were further added N-benzyloxycarbonyl-L-valine and dicyclohexylcarbodiimide in the same amounts, and they were stirred at room temperature for 48 hours. The precipitate was filtered from the reaction mixture, and the filtrate was concentrated under reduced pressure to obtain an oily substance. The oily substance was dissolved in 200 ml of ethyl acetate. The organic layer was washed once with 100 ml of a 5 % potassium hydrogensulfate aqueous solution and twice with 100 ml of a saturated NaCl aqueous solution, dried over anhydrous magnesium sulfate, and concentrated under reduced pressure to obtain an oily substance. The oily substance was analyzed by liquid chromatography. Consequently, 2-[(2-acetylamino-1,6-dihydro-6-oxo-9H-purin-9-yl)methoxy]ethyl N-[(benzyloxy)carbonyl]-L-valinate was obtained in a yield of 45.7 %.

The oily substance was dissolved in 40 ml of ethanol, and 4.05 g of triethylamine were added thereto, followed by refluxing the mixed solution for 7 hours. The reaction mixture was cooled to 0°C, and the precipitated crystals were filtered and washed with a small amount of ethanol. The thus-obtained crystals were dried at 50°C under reduced pressure to obtain 2.56 g of crystals of 2-[(2-amino-1,6-dihydro-6-oxo-9H-purin-9-yl)methoxy]ethyl N-[(benzyloxy)carbonyl]-L-valinate. The yield of this compound from 9-[(2-hydroxyethoxy)methyl]-N²-acetylguanine was 44.9 %.

### Example 4

### Synthesis of 2-[(2-acetylamino-1,6-dihydro-6-oxo-9H-purin-9-yl)methoxy]ethyl N-[(benzyloxy)carbonyl]-L-valinate from 9-[(2-hydroxyethoxy)methyl]-N²-acetylguanine:

12.5 ml of dimethylformamide were added to 1.34 g of 9-[(2-hydroxyethoxy)methyl]-N²-acetylguanine, and the mixture was cooled to 0 C. To the mixture were added 1.26 g of N-benzyloxycarbonyl-L-valine, 61 mg of 4-dimethylaminopyridine and 1.15 g of dicyclohexylcarbodiimide. The thus-obtained mixture was stirred at 0 C for 24 hours. To the reaction mixture were further added 0.25 g of N-benzyloxycarbonyl-L-valine, 12 mg of 4-dimethylaminopyridine and 0.10 g of dicyclohexylcarbodiimide, followed by stirring them at 0 C for 24 hours. The precipitate was filtered from the reaction mixture. The filtrate was analyzed by liquid chromatography. Consequently, 2-[(2-acetylamino-1,6-dihydro-6-oxo-9H-purin-9-yl)methoxy]ethyl N-[(benzyloxy)carbonyl]-L-valinate was obtained in a yield of 97.4 %. The filtrate was concentrated under reduced pressure, and the obtained oily substance was dissolved in 100 ml of ethyl acetate and washed with 50 ml of 5% potassium hydrogensulfate aqueous solution and with 50 ml x 2 of a saturated NaCl aqueous solution. 2-[(2-Acetylamino-1,6-dihydro-6-oxo-9H-purin-9-yl)methoxy]ethyl N-[(benzyloxy)carbonyl]-L-valinate was obtained in a yield of 93.9 % in this organic layer. The organic layer was allowed to stand at room temperature to precipitate white crystals. Tha crystals were filtered and dried to give 1.13 g of 2-[(2-acetylamino-1,6-dihydro-6-oxo-9H-purin-9-yl)methoxy]ethyl N-[(benzyloxy)carbonyl]-L-valinate in a yield of 42.7 %. Purity of the crystals was 94.6 %, and no dicyclohexylurea was detected.

### Example 5

### Synthesis of 2-[(2-amino-1,6-dihydro-6-oxo-9H-purin-9-yl)methoxy]ethyl N-[(benzyloxy)carbonyl]-L-valinate and 2-[(2-acetylamino-1,6-dihydro-6-oxo-9H-purin-9-yl)methoxy]ethyl N-[(benzyloxy)carbonyl]-L-valinate from 9- [(2-hydroxyethoxy)methyl]-N²-acetylguanine:

6.25 milliliters of dimethylformamide were added to 1.34 g of 9-[(2-hydroxyethoxy)methyl]-N²-acetylguanine, and the mixture was cooled to 0°C. To the mixture were added 1.26 g of N-benzyloxycarbonyl-L-valine, 30 mg of 4-dimethylaminopyridine and 1.14 g of dicyclohexylcarbodiimide. The mixture was stirred at 0°C for 24 hours. To the reaction mixture were further added 0.25 g of N-benzyloxycarbonyl-L-valine and 6.5 mg of 4-dimethylaminopyridine and 0.23 g of dicyclohexylcarbodiimide followed by stirring them at 0°C for 24 hours. The precipitate was filtered from the reaction mixture. The filtrate was analyzed by liquid chromatography. Consequently, 2-[(2-acetylamino-1,6-dihydro-6-oxo-9H-purin-9-yl)methoxy]ethyl N-[(benzyloxy)carbonyl]-L-valinate was obtained in a yield of 98.9 %. To the filtrate, 80 ml of ethylacetate was added and was washed with 50 ml of 5% potassium hydrogensulfate aqueous solution and with 50 ml x 2 of a saturated NaCl aqueous solution. After the first aqueous layer was extracted with 50 ml of ethyl acetate, the organic layers were combined. 2-[(2-Acetylamino-1,6-dihydro-6-oxo-9H-purin-9-yl)methoxy]ethyl N-[(benzyloxy)carbonyl]-L-valinate was obtained in a yield of 98.1 % in this combined organic layer. The combined organic layer was concentrated under reduced pressure, and the residue was dissolved in 30 ml of ethanol, and 2.7 ml of triethylamine was added to the solution, followed by refluxing the mixed solution for 15 hours. The reaction mixture was cooled to room temperature to precipitate white crystals. The crystals were filtered and dried to give 2.00 g of 2-[(2-amino-1,6-dihydro-6-oxo-9H-purin-9-yl)methoxy]ethyl N-[(benzyloxy)carbonyl]-L-valinate. Purity of the crystals was 95.1 %, and 0.3 % of dicyclohexylurea was detected. The yield of this compound from 9-[(2-hydroxyethoxy)methyl]-N²-acetylguanine was 83.0 %.

### Example 6

### Synthesis of 2-[(2-amino-1,6-dihydro-6-oxo-9H-purin-9-yl)methoxy]ethyl N-[(benzyloxy)carbonyl]-L-valinate and 2-[(2-acetylamino-1,6-dihydro-6-oxo-9H-purin-9-yl)methoxy]ethyl N-[(benzyloxy)carbonyl]-L-valinate from 9- [(2-hydroxyethoxy)methyl]-N²-acetylguanine:

6.25 milliliters of dimethylformamide were added to 1.34 g of 9-[(2-hydroxyethoxy)methyl]-N²- acetylguanine, and the mixture was cooled to 0°C. To the mixture were added 1.26 g of N-benzyloxycarbonyl-L-valine, 6.1 mg of 4-dimethylaminopyridine and 1.14 g of dicyclohexylcarbodiimide. The mixture was stirred at 0°C for 24 hours. To the reaction mixture were further added 0.25 g of N-benzyloxycarbonyl-L-valine and 1.2 mg of 4-dimethylaminopyridine and 0.23 g of dicyclohexylcarbodiimide followed by stirring them at 0°C for 24 hours. The precipitate was filtered from the reaction mixture. The filtrate was analyzed by liquid chromatography. Consequently, 2-[(2-acetylamino-1,6-dihydro-6-oxo-9H-purin-9-yl)methoxy]ethyl N-[(benzyloxy)carbonyl]-L-valinate was obtained in a yield of 99.0 %. The filtrate was concentrated under reduced pressure, and the obtained oily substance was dissolved in 20 ml of ethanol, and 2.7 ml of triethylamine were added to the solution, followed by refluxing the mixed solution for 11 hours. This reaction mixture was allowed to stand at room temperature to precipitate white crystals. Tha crystals were filtered and dried to give 2.01 g of 2-[(2-amino-1,6-dihydro-6-oxo-9H-purin-9-yl)methoxy]ethyl N-[(benzyloxy)carbonyl]-L-valinate. Purity of the crystals was 95.0 %, and no dicyclohexylurea was detected. The yield of this compound from 9-[(2-hydroxyethoxy)methyl]-N²-acetylguanine was 83.3 %.

### Example 7

### Synthesis of 2-[(2-acetylamino-1,6-dihydro-6-oxo-9H-purin-9-yl)methoxy]-ethyl N-[(benzyloxy)carbonyl]-L-valinate from 9-[(2-hydroxyethoxy)methyl]-N²-acetylguanine:

37.5 milliliters of dimethylformamide were added to 8.46 g of 9-[(2-hydroxyethoxy)methyl]-N²-acetylguanine (purity 94.8 %), and the mixture was cooled to 2°C. To the mixture were added 7.54 g of N-benzyloxycarbonyl-L-valine, 37 mg of 4-dimethylaminopyridine and 6.88 g of dicyclohexylcarbodiimide. The mixture was stirred at 2°C for 24 hours. To the reaction mixture were further added 1.51 g of N-benzyloxycarbonyl-L-valine and 1.38 g of dicyclohexylcarbodiimide followed by stirring them at 2°C for 24 hours. The precipitate was filtered from the reaction mixture. The filtrate was analyzed by liquid chromatography. Consequently, 2-[(2-acetylamino-1,6-dihydro-6-oxo-9H-purin-9-yl)methoxy]ethyl N-[(benzyloxy)carbonyl]-L-valinate was obtained in a yield of 96.1 %.

### Example 8

### Solubility of NAcZVA, ZVA and dicyclohexylurea(DCU)

To a test tube, 2.5 ml of ethyl acetate and 2-[(2-acetylamino-1,6-dihydro-6-oxo-9H-purin-9-yl)methoxy]ethyl N-[(benzyloxy)carbonyl]-L-valinate were added, and the mixture was stirred at room temperature (20 - 25 C) for 12 hours. The mixture was filtered. The filtrate was analyzed by liquid chromatography and solubility was calculated. Solubilities of NAcZVA in methylene chloride, acetontrile and dimethylformamide were measured in the same manner as ethyl acetate. Solubilities of ZVA and DCU in ethyl acetate, methylene chloride, acetonitrile and dimethylformamide were measured in the same manner as NAcZVA.

**Table 1**

| | Solubility(gram/deciliter) | | |
|---|---|---|---|
| Solvent | NAcZVA | ZVA | DCU |
| Ethyl acetate | 0.54 | 0.04 | 0.03 |
| Methylene chloride | 52.7 | 0.07 | 0.17 |
| Acetonitrile | 9.6 | 0.22 | 0.02 |
| N,N-Dimethylformamide | >56 | 42.0 | 0.23 |

## Claims

1. A process for the production of a compound represented by formula (1) 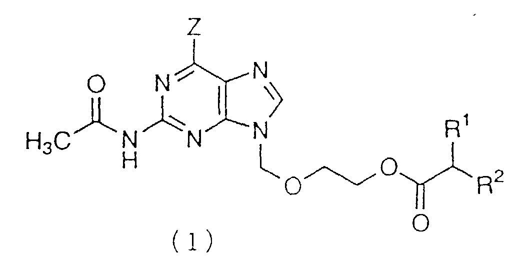 wherein Z denotes an optionally protected hydroxyl group, an optionally protected amino group, iodine, chlorine or hydrogen, R¹ denotes hydrogen, an alkyl group having from 1 to 10 carbon atoms, an aralkyl group, an optionally protected aminoalkyl group or an optionally protected carboxylalkyl group, and R² denotes an optionally protected amino group,
which comprises reacting in the presence of a suitable condensation agent a compound represented by formula (2) 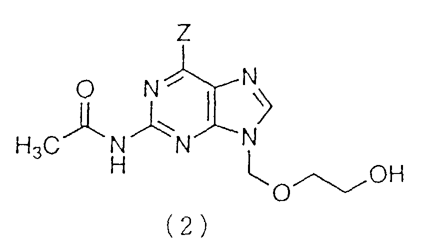 wherein Z denotes an optionally protected hydroxyl group, an optionally protected amino group, iodine, chlorine or hydrogen,
with a compound represented by formula (3) 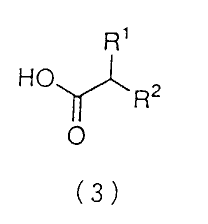 wherein R¹ denotes hydrogen, an alkyl group having from 1 to 10 carbon atoms, an aralkyl group, an optionally protected aminoalkyl group or an optionally protected carboxylalkyl group, and R² denotes an optionally protected amino group.

2. The process of claim 1 wherein the reaction is conducted in the presence of dicyclohexylcarbodiimide.

3. The process of claim 2 wherein dicyclohexylurea as a by-product formed in the reaction is removed by filtration.

4. The process of claim 1, 2 or 3 wherein Z in formula (2) is a hydroxyl group; the compound of formula (3) is N-benzyloxycarbonyl-L-valine; and in formula (1) R¹ is an isopropyl group, R² is a benzyloxycarbonylamino group, and the steric configuration of the carbon atom by which R¹ and R² are bound is S.

5. A compound represented by formula (1) 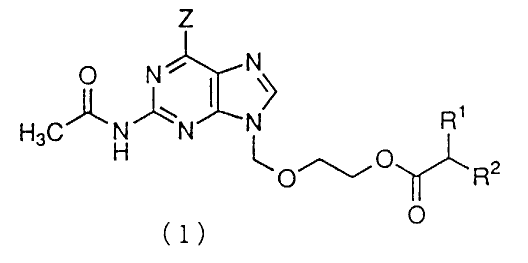 wherein Z is an optionally protected hydroxyl group, an optionally protected amino group, iodine, chlorine or hydrogen, R¹ denotes hydrogen, an alkyl group having from 1 to 10 carbon atoms, an aralkyl group, an optionally protected aminoalkyl group or an optionally protected carboxylalkyl group, and R² denotes an optionally protected amino group.

6. 2-[(2-Acetylamino-1,6-dihydro-6-oxo-9H-purin-9-yl)methoxy]ethyl N-benzyloxycarbonyl-L-valinate represented by formula (4).

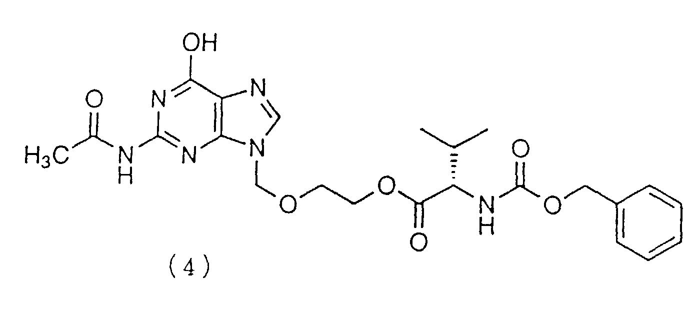

## Patentansprüche

1. Verfahren zur Herstellung einer durch die Formel (1) dargestellten Verbindung 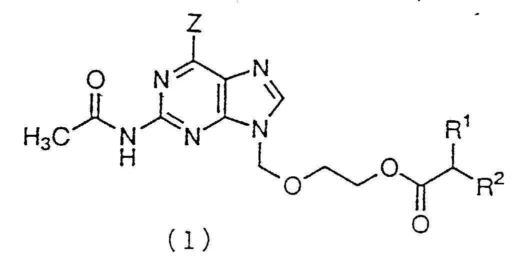 worin Z eine gegebenenfalls geschützte Hydroxygruppe, eine gegebenenfalls geschützte Aminogruppe, Iod, Chlor oder Wasserstoff darstellt, R¹ Wasserstoff, eine Alkylgruppe mit 1 bis 10 Kohlenstoffatomen, eine Aralkylgruppe, eine gegebenenfalls geschützte Aminoalkylgruppe oder eine gegebenenfalls geschützte Carboxyalkylgruppe darstellt und R² eine gegebenenfalls geschützte Aminogruppe darstellt,
welches das Umsetzen einer durch die Formel (2) dargestellten Verbindung 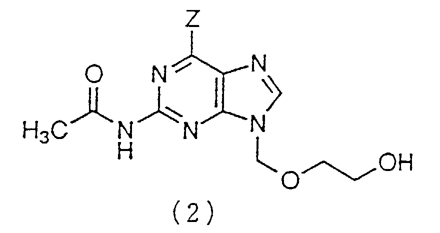 worin Z eine gegebenenfalls geschützte Hydroxygruppe, eine gegebenenfalls geschützte Aminogruppe, Iod, Chlor oder Wasserstoff darstellt,
mit einer durch die Formel (3) dargestellten Verbindung 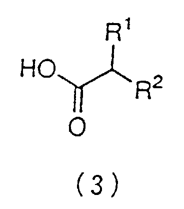 worin R¹ Wasserstoff, eine Alkylgruppe mit 1 bis 10 Kohlenstoffatomen, eine Aralkylgruppe, eine gegebenenfalls geschützte Aminoalkylgruppe oder eine gegebenenfalls geschützte Carboxyalkylgruppe darstellt, und R² eine gegebenenfalls geschützte Aminogruppe darstellt,
in Anwesenheit eines geeigneten Kondensationsmittels umfaßt.

2. Verfahren gemäß Anspruch 1, wobei die Reaktion in Anwesenheit von Dicyclohexylcarbodiimid durchgeführt wird.

3. Verfahren gemäß Anspruch 2, wobei Dicyclohexylharnstoff, das als Nebenprodukt in der Reaktion gebildet wird, durch Filtration entfernt wird.

4. Verfahren gemäß Anspruch 1, 2 oder 3, wobei Z in Formel (2) eine Hydroxygruppe ist, die Verbindung der Formel (3) N-Benzyloxycarbonyl-L-valin ist, und in der Formel (1) R¹ eine Isopropylgruppe, R² eine Benzyloxycarbonylaminogruppe und die sterische Konfiguration des Kohlenstoffatoms, an das R¹ und R² gebunden sind, S ist.

5. Durch die Formel (1) dargestellte Verbindung 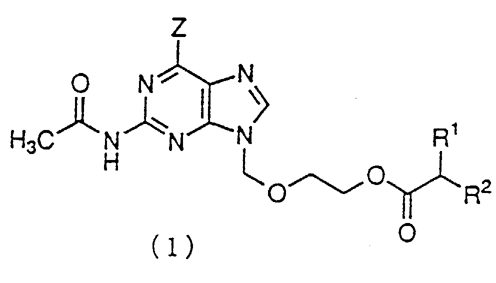 worin Z eine gegebenenfalls geschützte Hydroxygruppe, eine gegebenenfalls geschützte Aminogruppe, Iod, Chlor oder Wasserstoff ist, R¹ Wasserstoff, eine Alkylgruppe mit 1 bis 10 Kohlenstoffatomen, eine Aralkylgruppe, eine gegebenenfalls geschützte Aminoalkylgruppe oder eine gegebenenfalls geschützte Carboxyalkylgruppe darstellt, und R² eine gegebenenfalls geschützte Aminogruppe darstellt.

6. 2-[(2-Acetylamino-1,6-dihydro-6-oxo-9H-purin-9-yl)methoxy]ethyl-N-benzyloxycarbonyl-L-valinat, das durch die Formel (4) dargestellt ist.

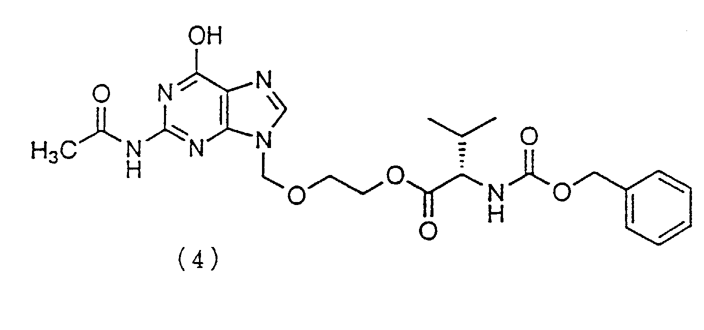

## Revendications

1. Procédé pour la production d'un composé représenté par la formule (1) 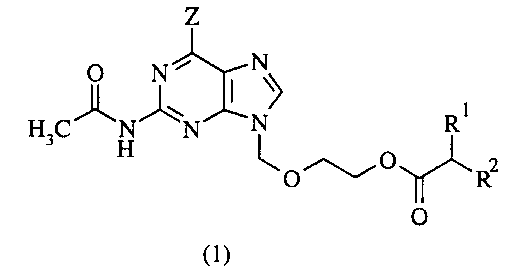 dans laquelle Z désigne un groupe hydroxyle éventuellement protégé, un groupe amino éventuellement protégé, un iode, un chlore ou un hydrogène, R¹ désigne un hydrogène, un groupe alkyle ayant 1 à 10 atomes de carbone, un groupe arylalkyle, un groupe aminoalkyle éventuellement protégé ou un groupe carboxyalkyle éventuellement protégé et R² désigne un groupe amino éventuellement protégé,
comprenant la réaction, en présence d'un agent de condensation approprié, d'un composé représenté par la formule (2) 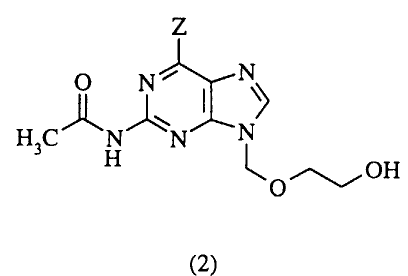 dans laquelle Z désigne un groupe hydroxyle éventuellement protégé, un groupe amino éventuellement protégé, un iode, un chlore ou un hydrogène, avec un composé représenté par la formule (3) 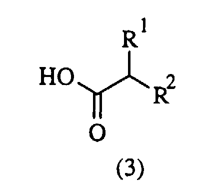 dans laquelle R¹ désigne un hydrogène, un groupe alkyle ayant 1 à 10 atomes de carbone, un groupe arylalkyle, un groupe aminoalkyle éventuellement protégé ou un groupe carboxyalkyle éventuellement protégé et R² désigne un groupe amino éventuellement protégé.

2. Procédé selon la revendication 1, dans lequel la réaction est conduite en présence de dicyclohexylcarbodiimide.

3. Procédé selon la revendication 2, dans lequel la dicyclohexylurée, sous-produit se formant au cours de la réaction, est éliminée par filtration.

4. Procédé selon la revendication 1, 2 ou 3, dans lequel Z dans la formule (2) est le groupe hydroxyle ; le composé de formule (3) est la N-benzyloxycarbonyl-L-valine et dans la formule (1) R¹ est le groupe isopropyle, R² est le groupe benzyloxycarbonylamino et la configuration stérique de l'atome de carbone auxquels sont liés R¹ et R² est S.

5. Composé représenté par la formule (1) 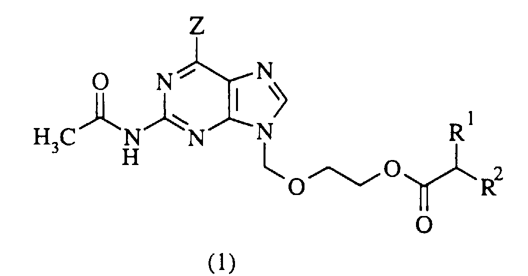 dans laquelle Z désigne un groupe hydroxyle éventuellement protégé, un groupe amino éventuellement protégé, un iode, un chlore ou un hydrogène, R¹ désigne un hydrogène, un groupe alkyle ayant 1 à 10 atomes de carbone, un groupe arylalkyle, un groupe aminoalkyle éventuellement protégé ou un groupe carboxyalkyle éventuellement protégé et R² désigne un groupe amino éventuellement protégé.

6. N-Benzyloxycarbonyl-L-valinate de 2-[(2-acétylamino-1,6-dihydro-6-oxo-9H-purin-9-yl)méthoxy]éthyle représenté par la formule (4)
